# FASCICULE DE BREVET EUROPEEN

(11) **EP 0 222 722 B1**
(45) Date de publication et mention de la délivrance du brevet: **19.05.1993**
(21) Numéro de dépôt: 86870042.8
(22) Date de dépôt: 08.04.1986
(51) Int. Cl.: C07K 15/00, A61K 37/02, A61K 39/395, A61K 47/00, A61K 31/475, C07D 519/04

(54) **Nouveaux conjugués de la vinblastine et de ses dérivés, procédé pour leur préparation et compositions pharmaceutiques contenant ces conjugués**
Konjugate von Vinblastin und seine Derivate, Verfahren zur Herstellung derselben und diese Konjugate enthaltende pharmazeutische Zusammensetzungen
Conjugates of vinblastine and its derivatives, process for preparing them and pharmaceutical compositions containing these conjugates

(30) Priorité: 12.11.1985 LU 86157
(43) Date de publication de la demande: 20.05.1987
(73) Titulaire: LA REGION WALLONNE, B-1050 Bruxelles (BE)
(72) Inventeur: Trouet, André Benoît Léon, B-3009 Winksele (BE); Rao, Kandukuri Sivaprasade Bushana, B-1331 Rosieres (BE); Hannart, Jean-Alfred Alphonse, B-1302 Dion-Valmont (BE); DeJonghe, Jean-Paul, B-1350 Wavre (BE)
(74) Mandataire: Van Malderen, Michel

(56) Documents cités:
- EP-A- 124 502
- EP-A- 0 121 388
- EP-A- 0 153 151

## Description

La présente invention se rapporte à de nouveaux conjugués de la vinblastine et de certains de ses dérivés connus avec des protéines ou des fragments de protéines servant d'agent anti-tumoral. L'invention s'étend également à un procédé pour les préparer et à des compositions pharmaceutiques contenant ces conjugués.

La vinblastine et certains de ses dérivés, en particulier la vincristine ou la vindésine, ont déjà été couplés à des protéines par exemple l'albumine ou diverses immunoglobulines. Il en résulte des produits de couplage ou des composés dits "conjugués". Nous relevons notamment les références suivantes de la littérature :
- J.D.Teale, Jacqueline M.Clough et V.Marks, Br.J.Clin.Pharmac. 4, 169-172 ,1977
- C.H.J.Ford, C.E.Newman, J.R.Johnson, C.S.Woodhouse, T.A.Reeder, G.F.Rowland, R.G.Simmonds, Br.J.Cancer 47, 35-42, 1983
- M.J.Embleton, G.F.Rowland, R.G.Simmonds, E.Jacobs, C.H.Marsden, R.W.Baldwin Br.J.Cancer 47,43-49, 1983
- J.R.Johnson, C.H.J.Ford, C.E.Newman, C.S.Woodhouse, G.F.Rowland, R.G.Simmonds Br.J.Cancer , 44, 472-475, 1981
- Eli Lilly Eur.Pat.Applic. , Publ. n°56.322, 21.07.82
- R.A. Conrad, G.J.Cullinan, K.Gerzon, G.A.Poore J.Med.Chem. 22, 391, 1979
On peut également citer la demande de brevet européen n° 84 870 057.1 dans laquelle on décrit le couplage de dérivés bis-indoliques à une protéine, un fragment de protéine ou une amine par l'intermédiaire d'un bras du type - COCH₂ -, -CO(CH₂)ₙ-CO, n variant de 1 à 5, qui peut être substitué par un groupe alkyle ou amino.

Le couplage de ces dérivés bis-indoliques a été entrepris non seulement dans le but de mettre au point de nouveaux réactifs immunologiques mais surtout en vue de la préparation de substances anti-tumorales plus actives ,plus sélectives et moins toxiques.

Dans cette dernière optique de nombreux conjugués protéiques avec d'autres agents antitumoraux sont actuellement étudiés. Il en est en particulier ainsi avec des conjugués d'anticorps et d'un fragment de la ricine ou de conjugués d'albumine et de méthotrexate (Demande de brevet français 2.437.213, C M Industries; B.C.F.Chu, S.B.Howell J. of Pharmacology and Exp. Therapeutics, 219 (2), 389-393, 1981).

Les anti-corps monoclonaux, en particulier ceux d'origine humaine, couplés aux médicaments anti-tumoraux connus sont plus particulièrement l'objet d'études diverses.

Le document EP-A- 121 388 est relatif à des conjugués d'alcaloïdes vinca avec des immunoglobulines en position C-4, présentant une activité dans le traitement du cancer.

Conformément à l'enseignement de ce document, l'immunoglobuline est liée au dérivé vinca via un groupe du type -COXCO- dans lequel X représente un lien chimique ou une chaîne C1-10 éventuellement substituée.

Le document EP-A-153 151 décrit de manière similaire des conjugués d'alcaloïdes vinca avec des transferrines.

Le document EP-A-124 502 est relatif à des conjugués de vinblastine et de ses dérivés, notamment avec des protéines ou des fragments de protéines dans lesquelles le couplage est effectué par l'intermédiaire d'un groupe ester dérivé du groupe hydroxyle du carbone 4 du squelette de la vinblastine.

Le couplage de la protéine ou du fragment de protéine au composé de type vinblastine est réalisé par l'intermédiaire d'un bras, par condensation préalable du dérivé de la vinblastine avec un dérivé organique. L'exemple 9 de la demande EP-A-124 502 telle que publiée décrit un composé de ce type pour lequel une activité anti-tumorale a été démontrée, et dans lequel le couplage du dérivé de vinblastine à l'albumine humaine galactosylée est réalisé par un bras hémisuccinate.

On relèvera enfin que l'utilisation et l'évaluation de complexes 1:1 d'alcaloïdes bis-indoliques avec la tubuline a été décrite dans le brevet belge 854.053. Dans certains cas, il peut en résulter une toxicité moindre et une activité chimiothérapique plus importante que celles des alcaloïdes libres correspondants.

La présente invention concerne des produits nouveaux qui sont des conjugués de la vinblastine ou de dérivés de la vinblastine avec des protéines ou des fragments de protéines, caractérisés en ce que le couplage est effectué par l'intermédiaire d'un groupe ester dérivé du groupe hydroxyle du carbone 4 du squelette de la vinblastine.

Ces conjugués suivant l'invention sont caractérisés en ce que la protéine, le fragment de protéine est couplé au composé de type vinblastine par l'intermédiaire d'un bras, par condensation préalable du dérivé de la vinblastine avec un dérivé organique bifonctionnel, éventuellement activé, selon une forme d'exécution préférée.

Le dérivé de la 0-4-désacétylvinblastine peut être par exemple la vindésine, la 0-4-désacétylvinblastine couplée en C-3 avec un acide aminé, la 0-4-désacétyldésoxy-4'-vinblastine ou la 0-4-désacétyldésoxy-4'-vinblastine couplée en C-3 avec un acide aminé.

Les composés de l'invention répondent plus particulièrement à la formule générale suivante 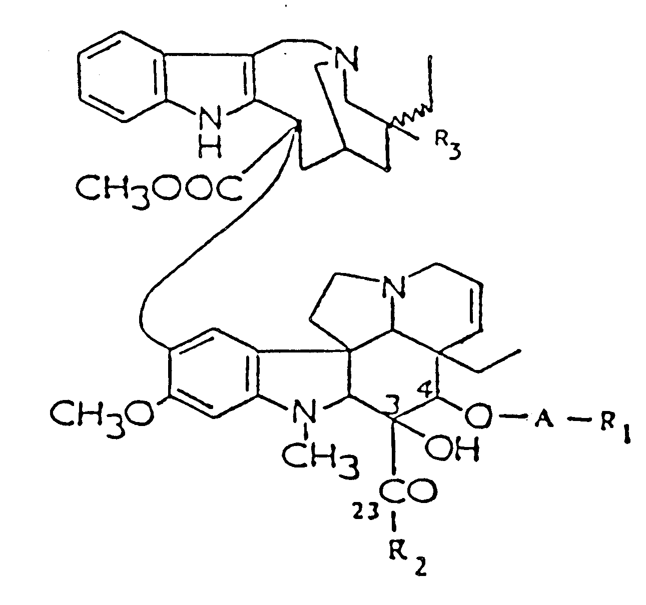
dans laquelle A représente un "bras"du type 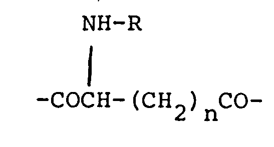
n variant de 1 à 5 et R représentant
un groupe acyle tel que acétyle, trifluoroacétyle ou carbobenzyloxy, R₁ représente un radical protéique éventuellement modifié par une galactosylation, R₂ représente un groupe méthoxy, un groupe amino ou un radical ester d'acide alpha-aminé lié par une liaison de type amide et dont le groupe ester comporte de 1 à 6 atomes de carbone, R₃ représente un atome d'hydrogène ou un groupe hydroxyle, chaque fois dans les deux configurations possibles ainsi que leurs sels d'addition avec un acide minéral ou organique.

L'activité thérapeutique des composés de l'invention est supérieure à celle des composés décrits jusqu'à présent et sa toxicité est sensiblement inférieure.

Les dérivés de la présente invention sont obtenus, dans une première étape, par condensation d'un anhydride, par exemple l'anhydride aspartique ou glutamique ou homologue supérieur, éventuellement substitués sur le groupement amine, sur l'hydroxyle en C-4 de la O-4-désacétylbinblastine ou de la O-4-désacétyldésoxyvinblastine ou l'un des dérivés du type O-4-désacétylvinblastine-carboxamide -3 ou O-4-desacétyldésoxyvinblastine-carboxamide-3.

Les dérivés hémiaspartate ou hémiglutamate ou homologues supérieurs ainsi obtenus sont alors condensés avec la protéine, le fragment de protéine dans un solvant dans lequel ces composés sont solubles. On peut à cette fin utiliser un mélange eau-dioxanne à un pH adéquat maintenu par un tampon, par exemple un tampon phosphate. La condensation est confirmée par chromatographie ou électrophorèse. Dans ce dernier cas on peut utiliser de la vinblastine radio-active (par exemple tritiée) afin de faciliter la caractérisation.

Du point de vue chimique la condensation avec les protéines peut s'expliquer par l'obtention de liens covalents résultant de la réaction des groupes amines de la lysine protéique avec le groupe ester activé dérivé de l'hémiaspartate ou hémiglutamate, éventuellement substitué.

L'albumine de sérum bovin par exemple comprend 56 résidus aminés de lysine. Le nombre de molécule de vinblastine par protéine variera en fonction des conditions opératoires mais sera généralement compris entre 1 et 34.

L'activation peut être effectuée de manière classique par traitement avec un chloroformiate d'alkyle, de préférence le chloroformiate d'éthyle ou d'isobutyle, en présence d'une base aminée telle la N-méthyl pipéridine ou la N-méthyl morpholine.

La condensation peut être effectuée in situ sur le mélange réactionnel contenant l'anhydride activé. Dans la plupart des cas l'anhydride activé peut cependant aussi être isolé.

Le conjugué obtenu est isolé au moyen de méthodes classiques utilisées en chimie ou, dans le cas de conjugués protéiques, en biochimie. Le conjugué protéique est ainsi précipité du milieu réactionnel par addition d'acétone, puis centrifugé, rincé, lyophilisé et purifié par filtration sur gel. Eventuellement on peut soumettre le dérivé ainsi obtenu à une réaction de succinylation classique qui permet d'éviter des problèmes d'agrégation que caractérisent certaines protéines, conjuguées ou non.

Les protéines qui peuvent être avantageusement utilisées sont en particulier l'albumine de sérum bovin ou humain, la fétuine ou des immunoglobulines, ces dernières étant éventuellement obtenues par la technique des anti-corps monoclonaux. Dans ce dernier cas, l'utilisation d'anti-corps monoclonaux d'origine humaine et montrant une certaine spécificité vis à vis de tumeurs humaines s'avère particulièrement intéressante.

Les protéines utilisées peuvent également être traitées afin d'être sélectivement modifiées. Ces modifications permettent d'obtenir des conjugués protéiques qui seront, lors de leur utilisation thérapeutique préférentiellement concentrées au niveau de certains tissus, par exemple au niveau du foie. Il est ainsi possible, préalablement à la condensation du dérivé de la vinblastine à la protéine, de galactosyler cette dernière. La galactosylation est effectuée en appliquant par exemple le mode opératoire décrit par G.Wilson dans The Journal of Biochemistry , 253 (7) 2070-2072, 1978.

Les essais in-vitro effectués avec les composés de l'invention pour démontrer leur activité anti-tumorale indiquent que la formation du conjugué par un lien en C-4 peut être plus avantageuse que lorsque ce lien est effectué en C-3.

En variante, on peut également condenser un composé du type -A-R₁, A et R₁ ayant les significations susmentionnées dans un solvant adéquat sur l'hydroxyle en C-4 de la O-4-désacétylvinblastine ou de la O-4-désacétyldésoxyvinblastine ou de l'un des dérivés du type O-4-désacétylvinblastine-carboxamide-3 ou O-4-désacétyldésoxyvinblastine-carboxamide-3.

L'activation s'effectue avantageusement par traitement avec un chloroformiate d'alkyle tel que le chloroformate d'éthyle ou d'isobutyle, en présence d'une base aminée telle que la N-méthyl-pipéridine ou la N-méthyl-morpholine ou la triéthylamine.

Les exemples suivants décrivent quelques conjugués selon la présente invention dans lesquels les dérivés bisindoliques sont couplés, en position C-4 à une protéine, ou un fragment de protéine, moyennant un bras du type hemiaspartate substitué ou hémiglutamate substitué.

La présente invention s'étend également aux applications industrielles et notamment pharmaceutiques des composés bisindoliques nouveaux.

Les composés de l'invention présentent en effet des propriétés antitumorales particulièrement intéressantes et susceptibles d'être utilisées en thérapeutique humaine.

Ces dérivés de la vinblastine peuvent être , en particulier, utilisés pour le traitement des leucémies, de gliomes, de lymphosarcomes ou d'autres tumeurs malignes, y compris les tumeurs dites "solides".

En thérapeutique humaine , ils sont ainsi utilisés pour le traitement de la maladie de Hodgkin et pour d'autres tumeurs pouvant bénéficier d'un traitement avec la vinblastine, la vincristine ou la vindésine.

Pour leur application thérapeutique, les composés de l'invention, éventuellement sous forme lyophilisée, sont de préférence administrés par voie parentérale, dissous dans un solvant pharmaceutiquement acceptable.

L'eau physiologique ou d'autres solutions salines tamponnées, par exemple avec un phosphate, sont des solvants appropriés.

La substance active est généralement administrée à une posologie pouvant varier de 50 mg à plusieurs grammes . Les composés de l'invention peuvent en outre être utilisés en combinaison avec d'autres agents anti-tumoraux.

### Exemple 1.

### a) Isomères α et β de la VBL-4-O-désacétyl-4-O-L-N-acétyl-hémiaspartate.

A une solution de 700 mg (0,91 mmol) de 0-4 déacétylvinblastine dans 20 ml de dichlorométhane anhydre, on ajoute 250 mg d'anhydride N-acetyl-L-aspartique.
La solution est agitée 20 heures à température ambiante. Après distillation du solvant sous vide, le résidu obtenu est purifié par chromatographie sur colonne de silice (élution éther/méthanol/NH₄OH 25% 60/49,75/0,25).
Après trituration dans un mélange de dichlorométhane et d'éther de pétrole, on obtient 650 mg du produit du titre sous forme d'une poudre blanche (rdt : 77%).
L'analyse du produit par HPLC indique la présence des deux isomères α et β dans un rapport 85/15.
* Spectre IR (KBr) : 3420, 2960, 2880, 1737, 1660, 1613, 1501, 1460, 1430 cm⁻¹.
* Spectre de masse : DCI (isobutane) : 925 (M⁺), 939 (M⁺ + 14), 857, 769, 693, 635.

### b) Isomères α et β de l'ester méthylique de la VBL-4-O-L-N-acétyl-hémiaspartate.

Une solution de 500 mg (0,540 mmoles) des isomères α et β de la VBL-4-N-acétyl-L-hémiaspartate dans 10 ml de méthanol absolu saturé avec de l'HCl sec est agitée 20 heures à température ambiante.
Après distillation du solvant sous vide, le résidu est repris par un mélange de 15 ml d'eau distillée et de 15 ml de dichlorométhane. Le mélange est alcalinisé par addition de NH₄OH. La phase aqueuse est extraite avec 3 portions de 20 ml de dichlorométhane. Les extraits organiques sont combinés, lavés successivement par 40 ml d'eau et par 40 ml d'une solution aqueuse saturée en NaCl, séchés sur du sulfate de magnesium et évaporés sous pression réduite. Le résidu est purifié par chromatographie sur silice (élution par CH₂Cl₂ : CH₃OH 95:5). On obtient de cette façon 410 mg des isomères α et β du produit du titre.
Rdt : 81%
Des fractions de chromatographie contenant chacune des isomères séparément ont été analysées.

### - Isomère principal.

* Spectre de masse (DCI, isobutane) : 939 (M⁺), 940 (M⁺ + 1), 953 (M⁺ + 14).
* Spectre IR (KBr) : 2950, 2880, 1740, 1675, 1615, 1503 cm⁻¹
* Spectre RMN (CDCl₃, 360 MHz)δ : 9,65 (OH), 8,02 (NH), 7,52 (H-9'), 7,16-7,05 (H-10', H-11', H-12'), 6,61 (H-9), 6,52 (NH), 6,07 (H-12), 5,75 (H-14), 5,52 (H-17), 5,17 (H-15), 4,75 (-CH-NH), 3,95 (H-17A'), 3,80 (-OMe), 3,77 (-OMe), 3,70 (-OMe), 3,60 (-OMe), 2,80 (H-21A', H-21B'), 2,67 (NMe), 2,62 (H-21), 2,00 (MeCO), 0,92-0,75 (2 Me)
* Rf : 0,51 (CH₂ Cl₂:CH₃OH 90:10 silice)

### - Isomère mineur.

* Spectre MS (DCI - isobutane) : 939 (M⁺), 940 (M⁺ +1).
* Spectre IR (KBr) : 1740, 1675, 1615 cm⁻¹.
* Spectre RMN (CDCl₃, 360 MHz)δ : 9.25 (OH), 8.02 (NH), 7.50 (H-9'), 7.16-7.05 (H-10', H-11', H-12'), 6.57 (H-9), 6.52 (NH), 6.08 (H-12), 5.82 (H-14), 5.47 (H-17), 5.28 (H-15), 4.75 (CH-NHAc), 3.93 (H-17A'), 3.77 (2 x OMe), 3.70 (OMe), 3.60 (OMe), 2.70 (N-Me), 2.02 (MeCO-), 0.95-0.77 (2 Me).
* Rf : 0,39 (CH₂Cl₂:CH₃OH 90:10 silice).

### Exemple 2.

### Isomères α et β de la VBL-4-O-désacétyl-4-O-L-N-carboxybenzyloxyhémiglutamate.

Une solution dans le dichlorométhane (5 ml) de 0-4 deacétylvinblastine (300 mg, 0,39 mmole), d'anhydride N-carbobenzyloxy-L-glutamique (144 mg, 0,519 mmole) est agitée 20 heures à température ambiante.
Le solvant est évaporé sous vide.
Le résidu obtenu est purifié par chromatographie sur colonne de silice (élution ether/methanol/NH₄OH 25% 50/49.5/0.5).
On obtient de cette façon 230 mg du produit du titre sous forme d'un mélange d'isomères α et γ.

L'analyse du produit par HPLC indique la présence des isomères α et γ dans un rapport 60:40.
* Spectre IR (KBr) : 3450, 2960, 2880, 1730, 1612, 1593, 1501, 1459, 1432, 1228 cm⁻¹.
* Spectre de masse (DCI - isobutane) : 1032 (M⁺ +1), 984, 928, 723 cm⁻¹

### Exemple 3 : Couplage de la VBL-4-O-désacétyl-4-O-L-N-acétyl hémiaspartate à l'albumine humaine galactosylée.

a) 80,6 mg de VBL-4-O-désacétyl-4-O-L-N-acétylhémiaspartate sont dissous dans 2 ml de dioxanne. La solution est ensuite plongée dans un bain de glace. On y ajoute 24,4 µl de triéthylamine dans 0,5 ml de dioxanne et 22,6 µl de chloroformiate d'isobutyle dans 0,5 ml de dioxanne. On agite pendant une heure. D'autre part, on prépare une solution de 200 mg d'albumine humaine galactosylée dans 37 ml d'eau. Le pH est ajusté à 8,5 avec de la soude 1N.
   La solution est refroidie à 4°C. Après 1 heure, l'ester activé est ajouté à la solution d'albumine humaine galactosylée. La solution est agitée pendant 14 heures à 4°C tandis que le pH est maintenu à 8,5 par addition de soude 1N. La solution est purifiée par filtration sur gel Sephadex G25 équilibré par une solution de NaCl 9/1000, pH 7,5. Les fractions contenant le conjugué sont rassemblées, concentrées par ultrafiltration et stérilisées. Le contenu en protéines est mesuré par la méthode de Lowry et le contenu en alcaloïdes estimé par mesure de la radioactivité.
   Le conjugué obtenu contient 13 moles de vinblastine par mole d'albumine humaine galactosylée. La détermination de la composition du conjugué en monomères, dimères et polymères, faite par HPLC, indique un pourcentage de 82 en monomères et 18 en dimères.
b) 80,6 mg de VBL-4-O-désacétyl-4-O-L-N-acétylhémiaspartate sont dissous dans 2 ml de dioxanne. La solution est ensuite plongée dans un bain de glace. On y ajoute 24,4 µl de triéthylamine dans 0,5 ml de dioxanne et 22,6 µl de chloroformiate d'isobutyle dans 0,5 ml de dioxanne. D'autre part, on prépare une solution de 200 mg d'albumine humaine galactosylée dans 37 ml de tampon phosphate 0,1 M, pH 8,2. Le pH est ajusté à 8,5 avec de la soude 1N.
   La solution est refroidie à 4°C. Après 1 heure, l'ester activé est ajouté à la solution d'albumine humaine galactosylée. La solution est agitée pendant 14 heures à 4°C tandis que le pH est maintenu à 8,5 par addition de soude 1N. La solution est purifiée par filtration sur gel Sephadex G25® équilibré par une solution de NaCl 9/1000, pH 7,5. Les fractions contenant le conjugué sont rassemblées, concentrées par ultrafiltration et stérilisées. Le contenu en protéines est mesuré par la méthode de Lowry et le contenu en alcaloïdes estimé par mesure de la radioactivité.
   Le conjugué obtenu contient 9,3 moles de vinblastine par mole d'albumine humaine galactosylée. La détermination de la composition du conjugué en monomères, dimères et polymères, faite par HPLC, indique un pourcentage de 91,5 en monomères, 7 en dimères et 1,5 en polymères.

La sensibilité du conjugué de l'exemple face aux enzymes lysosomiales a été étudiée par incubation, pendant 48 heures à 37°C, de ce conjugué, en présence de cystéine 5mM, de tampon acétate 40 mM et d'enzymes lysosomiales. Au cours du temps, des parties aliquotes sont prélevées et les protéines non dégradées sont précipitées par addition d'un volume d'acide trichloroacétique (TCA 40%). Après incubation des échantillons à 4°C pendant une heure, ceux-ci sont centrifugés et la radioactivité du surnageant est estimée par comptage d'une partie aliquote en scintillation liquide.

La radioactivité soluble est une mesure de la digestion du conjugué. L'expérience démontre que 75% du conjugué sont digérés après 24 heures. On n'observe plus d'évolution jusqu'à 48 heures.

L'activité chimiothérapeutique du conjugué obtenu selon l'exemple a été évaluée sur la leucémie P388 administrée par voie i.p. chez la souris BDF₁ femelle : 10⁶ cellules tumorales sont inoculées par voie i.p. au jour 0. Le conjugué est administré par voir i.p. au jour 1. Les résultats démontrent que le conjugué présente une activité très significative sur ce modèle expérimental puisqu'il induit une augmentation du temps de survie supérieure à 650% lorsqu'il est administré à la dose de 60 mg/kg et le nombre de survivants au jour 60 est de 5/5.

### Exemple 4

Isomères α et γ de N-(4-O-déacétyl-4-O-L-N acétylhémiaspartatevinblastinoyl-23)-L-tryptophanate d'éthyle.

En suivant la procédure de l'exemple 1, on a transformé le N-(déacétyl-O-4-vinblastinoyl-23)-L-tryptophanate d'éthyle en N-(4-O-déacétyl-4-O-N-acétyl-hémiaspartate-vinblastinoyl-23)-tryptophanate d'éthyle (mélange des isomères α et γ ).

Le résidu obtenu est purifié par chromatographie sur silice (élution = éther/méthanol/NH₄OH à 25% = 50/49,75/0,25).

On a obtenu 200 mg de produit à partir de 314 mg du produit de départ.

Spectre de masse : (DCl, Acétone) : 1126 (M⁺¹), 1066, 984, 970, 951, 911.
Spectre infrarouge: (KBr) : 3400, 2960, 2940, 1740, 1665, 1618 cm⁻

### Exemple 5

Isomères α et β de N-(4-O-déacétyl-4-O-L-N-acétyl-hémiaspartatevinblastinoyl-23)-L-isoleucinate d'éthyle.

Conformément à la procédure de l'exemple 4, on a transformé du N-(déacétyl-O-4-vinblastinoyl-23)-L-isoleucinate d'éthyle en N-(4-O-déacétyl-4-O-L-N-acétyl-hémiaspartate-vinblastinoyl-23)-L-isoleucinate d'éthyle.

Spectre de masse: (DCl-acétone): 1051 (M⁺),1036, 1009, 977, 897, 838, 755, 709, 652.
Spectre infrarouge (KBr): 3410, 2963, 2929, 2880, 1734, 1676, 1612, 1500, 1460, 1430, 1372, 1333, 1293 cm⁻¹.

### Exemple 6

Isomères α et γ de 4-O-déacétyl-4-O-L-N-acétylhémiglutamate vinblastine.

Conformément à la procédure de l'exemple 2, on a traité le O-4-déacétylvinblastine avec de l'anhydride N-acétyl-L-glutamique en vue de produire 271 mg de O-4-déacétyl-4-O-L-N-acétyl-hémiglutamate vinblastine (mélange des isomères α et γ ) à partir de 380 mg de O-4-déacétylvinblastine.

Spectre de masse (DCl-acétone) : 940(M⁺¹), 871, 707
Spectre infrarouge (KBr): 3470, 2960, 2880, 1740, 1665, 1617 cm⁻¹.

## Revendications (Revendications pour l'(les) Etat(s) contractant(s) suivant(s): BE, CH, DE, FR, GB, IT, LI, LU, NL, SE)

1. Conjugué de vinblastine ou d'un dérivé de celle-ci répondant à la formule générale 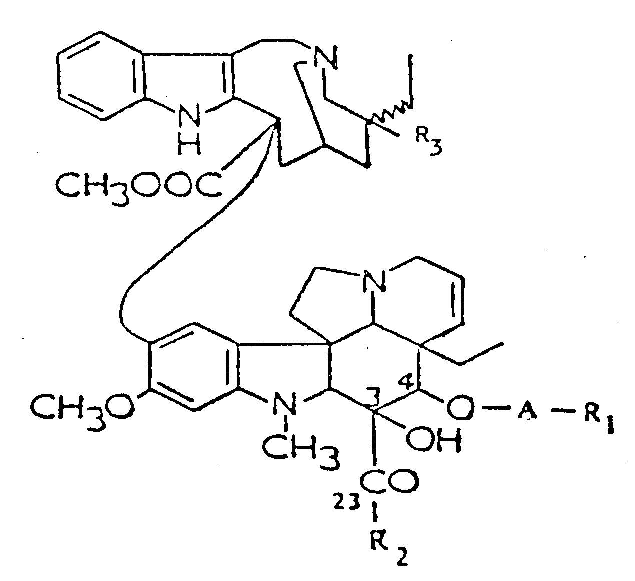 dans laquelle A représente un "bras" du type 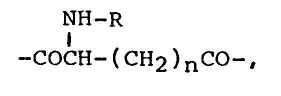 n variant de 1 à 5 et R représentant un groupe acyle tel que acétyle, trifluoroacétyle ou carbobenzyloxy, R₁ représente un radical protéique éventuellement modifie par une galactosylation, R₂ représente un groupe méthoxy, un groupe amino ou un radical ester d'acide alpha-aminé lié par une liaison de type amide et dont le groupe ester comporte de 1 à 6 atomes de carbone, R₃ représente un atome d'hydrogène ou un groupe hydroxyle, chaque fois dans les deux configurations possibles.

2. Conjugué selon la revendication 1 caractérisé en ce que le radical protéique est dérivé de l'albumine de sérum bovin ou humain, la fétuine ou des immunoglobulines éventuellement obtenues par la technique des anticorps monoclonaux, de préférence d'anticorps monoclonaux humains.

3. Conjugué selon la revendication 1 ou 2 caractérisé en ce que l'ester d'acide aminé est le tryptophanate d'éthyle ou l'isoleucinate d'éthyle.

4. Conjugué selon la revendication 1 ou 2 caractérisé en ce que R₂ représente un groupe methoxy ou amino.

5. Procédé de préparation de conjugués selon l'une quelconque des revendications 1 à 4 caractérisé en ce qu'on condense un anhydride d'acide du type 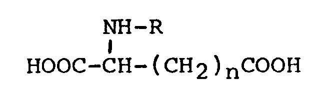 sur l'hydroxyle en position C-4 du dérivé de la vinblastine, et en ce qu'on condense ensuite le dérivé obtenu avec le radical protéique.

6. Procédé selon la revendication 5 caractérisé en ce que le solvant de la première condensation consiste essentiellement en du dichlorométhane et en ce que le solvant utilisé dans la deuxième étape de condensation est un mélange d'eau et de dioxanne.

7. Procédé de préparation de conjugués selon l'une quelconque des revendications 1 à 4 caractérisé en ce qu'on condense un composé du type 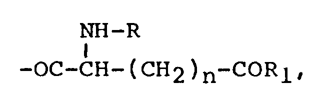 R₁ étant un radical protéique, sur l'hydroxyle en position C-4 du dérivé de la vinblastine.

8. Procédé selon la revendication 7 caractérisé en ce qu'on effectue une activation du composé de départ, par traitement avec un chloroformiate d'alkyle tel que le chloroformiate d'éthyle ou d'isobutyle, en présence d'une base aminée telle que la N-méthyl-pipéridine ou la N-méthyl-morpholine ou la triéthylamine.

9. Procédé selon l'une quelconque des revendications 5 à 8 caractérisé en ce qu'on effectue encore une centrifugation, un rinçage, une lyophilisation et/ou une purification par filtration sur gel, ainsi qu'éventuellement une succinylation classique.

10. Procédé selon l'une quelconque des revendications 5 à 9 caractérisé en ce que le radical protéique est dérivé de l'albumine de sérum bovin ou humain de la fétuine ou des immunoglobulines éventuellement obtenues par la technique des anticorps monoclonaux, de préférence d'anticorps monoclonaux humains, le radical protéique étant éventuellement traité afin d'être sélectivement modifié.

11. Composition pharmaceutique caractérisée en ce qu'elle comporte les conjugués selon l'une des revendications 1 à 4, en combinaison avec un diluant, solvant, excipient ou support pharmaceutiquement acceptable, le composé actif se trouvant sous forme lyophilisée ou en solution dans un solvant tamponné, pour le traitement des leucémies, de lymphosarcomes ou autres tumeurs malignes, y compris les tumeurs dites "solides" et pour le traitement de la maladie d'Hodgkin.

## Revendications (Revendications pour l'(les) Etat(s) contractant(s) suivant(s): AT)

1. Procédé pour la préparation de conjugués de vinblastine ou d'un dérivé de celle-ci répondant à la formule générale 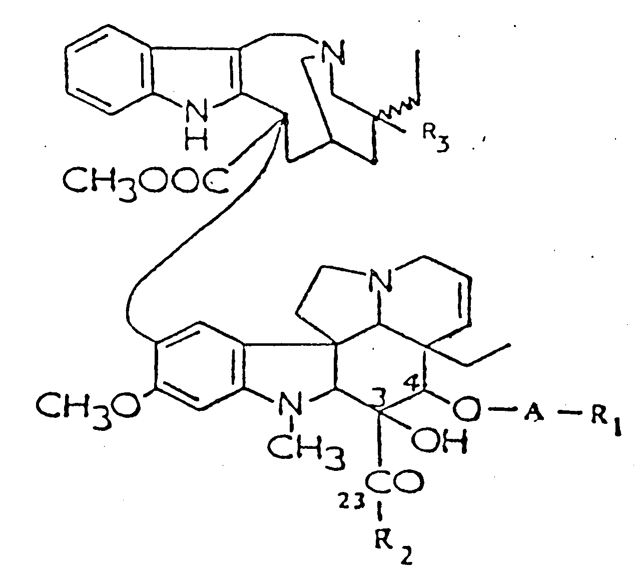 dans laquelle A représente un "bras" du type 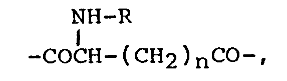 n variant de 1 à 5 et R représentant un groupe acyle tel que acétyle, trifluoroacétyle ou carbobenzyloxy, R₁ représente un radical protéique éventuellement modifié par une galactosylation, R₂ représente un groupe méthoxy, un groupe amino ou un radical ester d'acide alpha-aminé lié par une liaison de type amide et dont le groupe ester comporte de 1 à 6 atomes de carbone, R₃ représente un atome d'hydrogène ou un groupe hydroxyle, chaque fois dans les deux configurations possibles caractérisé en ce qu'on condense un anhydride d'acide du type 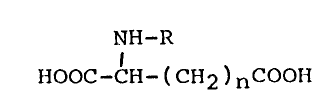 sur l'hydroxyle en C-4 du dérivé de vinblastine et en ce qu'on condense ensuite le dérivé ainsi obtenu avec ledit radical protéique; ou bien en ce qu'on condense un composé du type 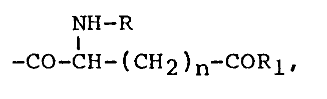 R₁ étant un radical protéique, sur l'hydroxyle en position C-4 du dérivé de la vinblastine.

2. Procédé selon la revendication 1 caractérisé en ce que le radical protéique est dérivé de l'albumine de sérum bovin ou humain, de la fétuine ou des immunoglobulines éventuellement obtenues par la technique des anticorps monoclonaux, de préférence les anticorps monoclonaux humains.

3. Procédé selon la revendication 1 ou 2 caractérisé en ce que, lors de la condensation d'un anhydride d'acide du type 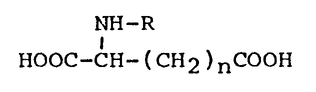 sur l'hydroxyle en C-4 du dérivé de la vinblastine, on utilise un solvant qui consiste essentiellement en du dichlorométhane et en ce que, lors de la condensation du produit ainsi obtenu avec le radical protéique, on utilise un solvant qui consiste essentiellement en un mélange d'eau et de dioxane.

4. Procédé selon la revdnciation 1 ou 2 caractérisé en ce que, lors de la condensation du produit du type -A-R₁ avec le dérivé de vinblastine en position C-4, on active celui-ci par traitement avec un chloroformiate d'alkyle tel que le chloroformiate d'éthyle ou d'isobutyle, en présence d'une base aminée telle que la N-méthyl-pipéridine ou la N-méthyl-morpholine ou la triéthylamine.

## Claims (Claims for the following Contracting State(s): BE, CH, LI, DE, FR, GB, IT, LU, NL, SE)

1. Conjugate of vinblastine or of a derivative thereof corresponding to the general formula: 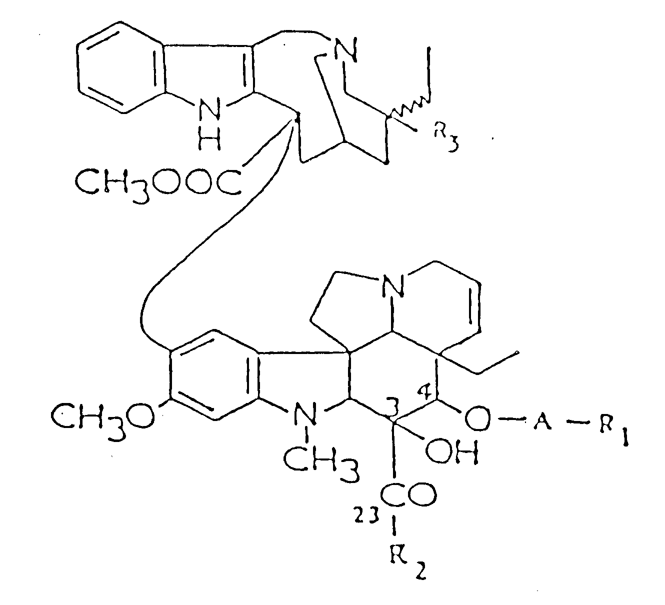 in which A represents an "arm" such as 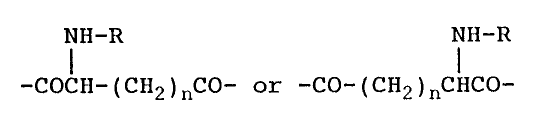 in which n varies from 1 to 5 and R represents a hydrogen atom or an acyl group such as acetyl, trifluoroacetyl or carbobenzyloxy by a galactosylation, R₂ represents a methoxy group, an amino group or an alpha-aminoacid ester radical which is bonded by a bond of the amide type and in which the ester group contains 1 to 6 carbon atoms, and R₃ represents a hydrogen atom or a hydroxyl group, in each case in the two possible configurations.

2. Conjugate according to claim 1, characterised in that the protein radical is derived from bovine or human serum albumin, fetuin or immunoglobulins, the latter being obtained, if appropriate, by the monoclonal antibody technique, preferably monoclonal antibodies of human origin.

3. Conjugate according to claim 1 or 2, characterised in that the aminoacid ester is ethyl tryptophanate or ethyl isoleucinate.

4. Conjugate according to claim 1 or 2, characterised in that R₂ represents a methoxy or amino group.

5. Process for the preparation of conjugates according to any of the claims 1 to 4, characterised in that an anhydride of an acid of the 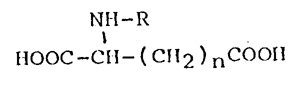 type is condensed onto the hydroxyl at C-4 of the vinblastine derivative and in that the derivative thus obtained is then condensed with the protein radical.

6. Process according to claim 5, characterised in that the solvent for the first condensation consists essentially of dicloromethane and in that the solvent used for the second condensation stage is a water/dioxane mixture.

7. Process for the preparation of conjugates according to any of claims 1 to 4, characterised in that a moiety of the type 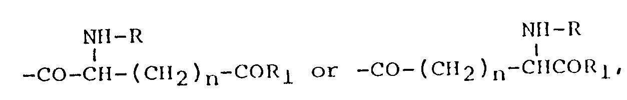 in which R₁ is a protein radical, is condensed onto the hydroxyl at C-4 of the vinblastine derivative.

8. Process according to claim 7, characterised in that an activation of the starting product is effected by treatment with an alkyl chloroformate, such as ethyl chloroformate, in the presence of an amine base, such as N-methylpiperidine or N-methylmorpholine or triethylamine.

9. Process according to any of claims 5 to 8, characterised in that a centrifugation, a rinsing, a lyophilisation and/or a purification by gel filtration are furthermore carried out, as well as optionnally a conventional succinylation reaction.

10. Process according to any of claims 5 to 9, characterised in that the protein radical is derived from bovine or human serum albumin, fetuin on immunoglobulins, the latter being obtained, if appropriate, by the monoclonal antibody technique, preferably monoclonal antibodies of human orogin, said protein radical being optionally treated in order to be selectively modified.

11. Pharmaceutical composition, characterised in that it comprises the conjugates according to any of claims 1 to 4, in combination with a diluent, a solvent, an excipient or a pharmaceutically acceptable support, the active compound being in lyophilised form or in solution in a buffered solvent, for the treatment of leukemias, lymphosarcomas or other malignant tumours, including so-called "solid" tumours and for the treatment of Hodgkin's disease.

## Claims (Claims for the following Contracting State(s): AT)

1. Process for the preparation of conjugates of vinblastine or of a derivative thereof corresponding to the general formula: 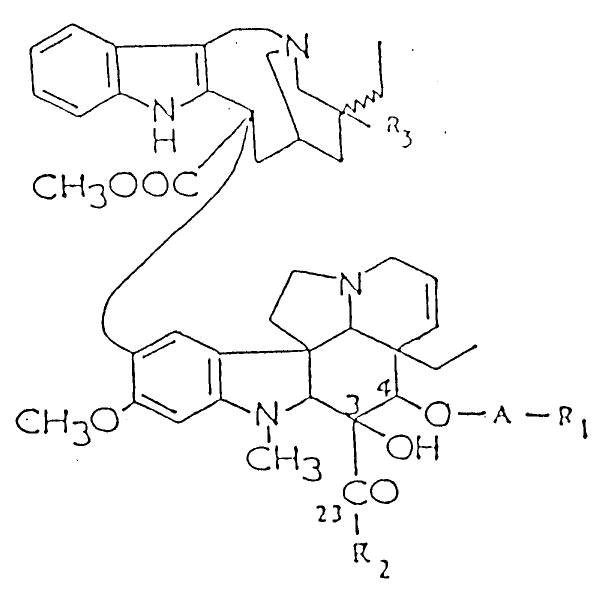 in which A represents an "arm" such as 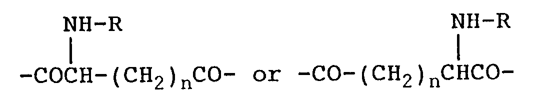 in which n varies from 1 to 5 and R represents a hydrogen atom or an acyl group such as acetyl, trifluoroacetyl or carbobenzyloxy by a galactosylation, R₂ represents a methoxy group, an amino group or an alpha-aminoacid ester radical which is bonded by a bond of the amide type and in which the ester group contains 1 to 6 carbon atoms, and R₃ represents a hydrogen atom or a hydroxyl group, in each case in the two possible configurations.

2. Process according to claim 1, characterised in that the protein radical is derived from bovine or human serum albumin, fetuin or immunoglobulins, the latter being obtained, if appropriate, by the monoclonal antibody technique, preferably monoclonal antibodies of human origin.

3. Process according to claim 1 or 2, characterized in that in the condensation of an anhydride of an acid of the type 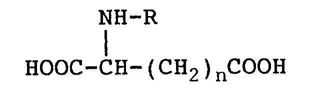 onto the hydroxyl at C-4 of the vinblastine derivative, the solvent used consists essentially of dicloromethane and in that for the condensation of the obtained product the solvent used is a water/dioxane mixture.

4. Process according to claims 1 or 2, characterized in that in the condensation of the product of the type -A-R, with the C-4 vinblastine derivative, the said product is activated by treatment of an alkyl chloroformate, such as ethyl chloroformate or isobutyl chloroformate, in the presence of an amine base, such as N-methylpiperidine or N-methylmorpholine or triethylamine.

## Patentansprüche (Patentansprüche für folgende(n) Vertragsstaat(en): BE, CH, DE, FR, GB, IT, LI, LU, NL, SE)

1. Konjugierte Verbindung aus Vinblastin oder einem Vinblastinderivat, die der allgemeinen Formel 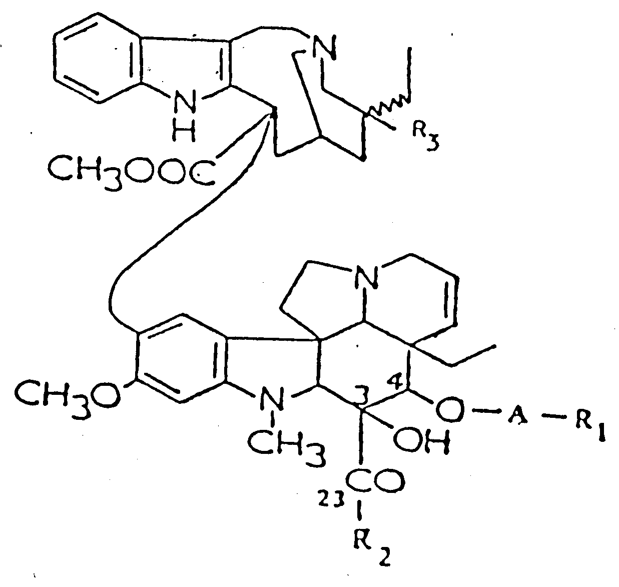 entspricht, in der A einen "Zweig" vom Typ 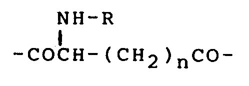 darstellt, wobei n von 1 bis 5 variiert, und R eine Acylgruppe, wie Acetyl, Trifluoracetyl oder Carbobenzyloxy darstellt, R₁ ein eventuell durch eine Galaktosylation modifiziertes Proteinradikal darstellt, R₂ eine Methoxygruppe, eine Aminogruppe oder ein Alphaaminosäureesterradikal darstellt, wobei dieses Radikal über eine Verbindung vom Amidtyp gebunden ist und seine Estergruppe 1 bis 6 Kohlenstoffatome aufweist, R₃ ein Wasserstoffatom oder eine Hydroxylgruppe darstellt, und zwar jedes Mal in den zwei möglichen Konfigurationen.

2. Konjugierte Verbindung gemäß Anspruch 1, dadurch gekennzeichnet, daß das Proteinradikal von Rinder- oder Humanserumalbumin, Fetuin oder Immunglobulinen abgeleitet ist, wobei diese Immunglobuline eventuell mittels der Technik der monoklonalen Antikörper, vorzugsweise aus menschlichen monoklonalen Antikörpern, erhalten werden.

3. Konjugierte Verbindung gemäß Anspruch 1 oder 2, dadurch gekennzeichnet, daß der Aminosäureester Äthyltryptophanat oder Äthylisoleucinat ist.

4. Konjugierte Verbindung gemäß Anspruch 1 oder 2, dadurch gekennzeichnet, daß R₂ eine Methoxy- oder Aminogruppe darstellt.

5. Verfahren zur Herstellung von konjugierten Verbindungen gemäß irgendeinem der Ansprüche 1 bis 4, dadurch gekennzeichnet, daß ein Säureanhydrid vom Typ 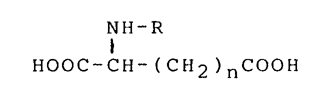 auf dem Hydroxyl in der Position C-4 des Vinblastinderivats kondensiert wird, und daß danach das erhaltene Derivat mit dem Proteinradikal kondensiert wird.

6. Verfahren gemäß Anspruch 5, dadurch gekennzeichnet, daß das Lösungsmittel der ersten Kondensation im wesentlichen aus Dichlormethan besteht, und daß das bei dem zweiten Kondensationsschritt verwendete Lösungsmittel ein Gemisch aus Wasser und Dioxan ist.

7. Verfahren zur Herstellung von konjugierten Verbindungen gemäß irgendeinem der Ansprüche 1 bis 4, dadurch gekennzeichnet, daß eine Verbindung vom Typ 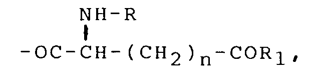 wobei R₁ ein Proteinradikal ist, auf dem Hydroxyl in der Position C-4 des Vinblastinderivats kondensiert wird.

8. Verfahren gemäß Anspruch 7, dadurch gekennzeichnet, daß eine Aktivierung der Ausgangsverbindung vorgenommen wird durch Behandlung mit einem Alkylchlorformiat, wie Äthyl- oder Isobutylchlorformiat, in Gegenwart eines primären Amins, wie N-Methyl-piperidin oder N-Methyl-morpholin oder Triäthylamin.

9. Verfahren gemäß irgendeinem der Ansprüche 5 bis 8, dadurch gekennzeichnet, daß noch eine Zentrifugierung, eine Spülung, eine Lyophilisierung und/oder eine Reinigung durch Gelfiltration, sowie eventuell eine herkömmliche Succinylation vorgenommen werden.

10. Verfahren gemäß irgendeinem der Ansprüche 5 bis 9, dadurch gekennzeichnet, daß das Proteinradikal von Rinder- oder Humanserumalbumin, Fetuin oder Immunglobulinen abgeleitet ist, wobei diese Immunglobuline eventuell mittels der Technik der monoklonalen Antikörper, vorzugsweise aus menschlichen monoklonalen Antikörpern, erhalten werden, und wobei das Proteinradikal eventuell behandelt wird, um es selektiv zu modifizieren.

11. Pharmazeutische Zusammensetzung, dadurch gekennzeichnet, daß sie die konjugierten Verbindungen gemäß einem der Ansprüche 1 bis 4 aufweist, in Kombination mit einem Verdünnungsmittel, Lösungsmittel, Exzipienten oder pharmazeutisch akzeptablen Träger, wobei die aktive Verbindung in lyophilisierter Form, oder in Lösung in einem gepufferten Lösungsmittel vorliegt, zur Behandlung von Leukämien, Lymphosarkomen oder anderen bösartigen Tumoren, einschließlich der sogenannten "festen" Tumore, und zur Behandlung der Hodgkin-Krankheit.

## Patentansprüche (Patentansprüche für folgende(n) Vertragsstaat(en): AT)

1. Verfahren zur Herstellung von konjugierten Verbindungen aus Vinblastin oder einem Vinblastinderivat, die der allgemeinen Formel 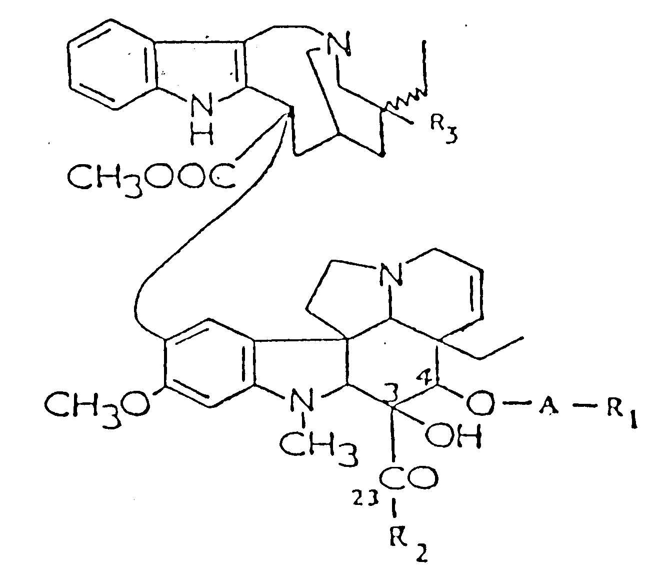 entsprechen, in der A einen "Zweig" vom Typ 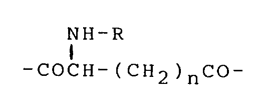 darstellt, wobei n von 1 bis 5 variiert, und R eine Acylgruppe, wie Acetyl, Trifluoracetyl oder Carbobenzyloxy darstellt, R₁ ein eventuell durch eine Galaktosylation modifiziertes Proteinradikal darstellt, R₂ eine Methoxygruppe, eine Aminogruppe oder ein Alphaaminosäureesterradikal darstellt, wobei dieses Radikal über eine Verbindung vom Amidtyp gebunden ist und seine Estergruppe 1 bis 6 Kohlenstoffatome aufweist, R₃ ein Wasserstoffatom oder eine Hydroxylgruppe darstellt, und zwar jedes Mal in den zwei möglichen Konfigurationen, dadurch gekennzeichnet, daß ein Säureanhydrid vom Typ 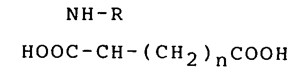 auf dem Hydroxyl bei C-4 des Vinblastinderivats kondensiert wird, und daß danach das so erhaltene Derivat mit diesem Proteinradikal kondensiert wird; oder daß eine Verbindung vom Typ 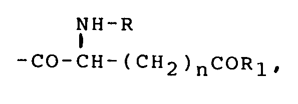 wobei R₁ ein Proteinradikal ist, auf dem Hydroxyl in der Position C-4 des Vinblastinderivats kondensiert wird.

2. Verfahren gemäß Anspruch 1, dadurch gekennzeichnet, daß das Proteinradikal von Rinder- oder Humanserumalbumin, Fetuin oder Immunglobulinen abgeleitet ist, wobei diese Immunglobuline eventuell mittels der Technik der monoklonalen Antikörper, vorzugsweise aus menschmenschlichen monoklonalen Antikörpern, erhalten werden.

3. Verfahren gemäß Anspruch 1 oder 2, dadurch gekennzeichnet, daß bei der Kondensation eines Säureanhydrids vom Typ 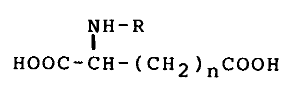 auf dem Hydroxyl bei C-4 des Vinblastinderivats ein Lösungsmittel verwendet wird, das im wesentlichen aus Dichlormethan besteht, und daß bei der Kondensation des so erhaltenen Produkts mit dem Proteinradikal ein Lösungsmittel verwendet wird, das im wesentlichen aus einem Gemisch aus Wasser und Dioxan besteht.

4. Verfahren gemäß Anspruch 1 oder 2, dadurch gekennzeichnet, daß bei der Kondensation des Produkts vom Typ -A-R₁ mit dem Vinblastinderivat in der Position C-4, dieses Produkt aktiviert wird durch Behandlung mit einem Chlorformiat, wie Äthyl- oder Isobutylchlorformiat, in Gegenwart eines primären Amins, wie N-Methylpiperidin oder N-Methyl-morpholin oder Triäthylamin.
